# EUROPÄISCHE PATENTSCHRIFT

(11) **EP 0 573 887 B1**
(45) Veröffentlichungstag und Bekanntmachung des Hinweises auf die Patenterteilung: **18.09.1996**
(21) Anmeldenummer: 93108847.0
(22) Anmeldetag: 02.06.1993
(51) Int. Cl.: C07D 301/12

(54) **Verfahren zur Epoxierung von Olefinen mit Peroxiden und Titanzeolithen**
Process for the epoxidation of olefins with peroxydes and titanzeolites
Procédé pour l'épxidation d'oléfines avec des péroxydes et des zéolites de titan

(30) Priorität: 06.06.1992 DE 4218765
(43) Veröffentlichungstag der Anmeldung: 15.12.1993
(73) Patentinhaber: BASF Aktiengesellschaft, 67063 Ludwigshafen (DE)
(72) Erfinder: Horchler von Locquenghien, Klaus, Dr., W-6708 Neuhofen (DE); Hoelderich, Wolfgang, Prof. Dr., W-6710 Frankenthal (DE); Oftring, Alfred, Dr., W-6702 Bad Duerkheim (DE)

(56) Entgegenhaltungen:
- EP-A- 0 100 119
- EP-A- 0 190 609
- EP-A- 0 492 697
- EP-A- 0 568 336
- EP-A- 0 568 337
- US-A- 3 651 136
- US-A- 3 923 843
- J.Catal, vol.130,(1991),p.1-8.
- J.C.S. Chem. Commun., 1994,p.1667-1668.
- J. Mol. Catal., 7(1980), 107-26

## Beschreibung

Die vorliegende Erfindung betrifft ein neues Verfahren zur Herstellung von funktionell substituierten Oxiranen aus den entsprechend substituierten Olefinen durch Umsetzung mit Wasserstoffperoxid und/oder organischen Peroxiden in Gegenwart von Titanzeolithen mit einer Kristallgröße von mehr als 1 µm und plättchenförmiger Kristallform bei erhöhten Temperaturen.

Die Herstellung von Oxiranen durch Epoxidierung geeigneter Olefine wird in der Regel mit peroxidischem Sauerstoff durchgeführt, der in Form von Wasserstoffperoxid, organischen Peroxiden oder Persäuren zum Einsatz kommen kann. Sie kann nach B. Notari, Stud. Surf. Sci. Catal., 37, Seite 413 bis 425 (1987) sowie EP-A-230 949 und EP-A-315 247 heterogenkatalytisch an Titanzeolithen als Katalysatoren durchgeführt werden.

Aus der EP-A-110 119 ist ein Verfahren bekannt, wonach Propen in wäßriger Phase mit Wasserstoffperoxid an Titanzeolithen zu Propylenoxid epoxidiert werden kann. Die Oxidation gesättigter Kohlenwasserstoffe von C₂- bis C₁₈- mit H₂O₂ an Titanzeolithen ist aus der EP-A-376 453 bekannt.

Die in J. Inst. Chem. (India) 1983, 55, S. 159 beschriebene Epoxidierung von Acrylsäure unter Verwendung eines 3A Zeolithen erscheint recht ungewöhnlich, da dieses Material nur als Wasserfänger fungieren kann.

In EP-A-190 609 sind u.a. Umsetzungen von Allylestern der Acryl- und Methacrylsäure an Titanzeolithen beschrieben. Dabei zeigte sich, daß unter den gewählten Reaktionsbedingungen selektiv die Allyldoppelbindung epoxidiert wird, während die Doppelbindung des Michaelsystems nicht angegriffen wird.

Aus der nicht vorveröffentlichten EP-A-568 336 (Artikel 54(3) EPÜ) ist ein Verfahren zur Epoxidierung an kristallinen Titansilikalit-Zeolithen mit einem Molverhältnis von Si:Ti von 8:1 bis 23:1 bekannt.

Der vorliegenden Erfindung lag daher die Aufgabe zugrunde ein Verfahren zu entwickeln um konjugierte Doppelbindungen (Michaelsysteme) zu epoxidieren.

Demgemäß wurde ein neues und verbessertes Verfahren zur Herstellung von Oxiranen der allgemeinen Formel I 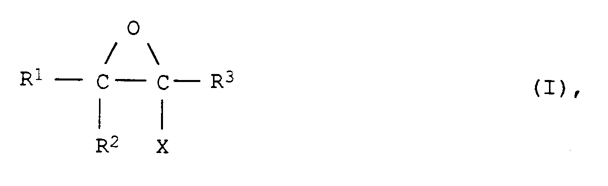 in der
- X: -COOH, -COOR⁴ oder -CN,
- R¹,R²,R³,R⁴: C₁- bis C₈-Alkyl, C₃- bis C₈-Cycloalkyl, Aryl und C₇- bis C₁₂-Aralkyl oder
- R¹,R²,R³: Wasserstoff bedeuten,
gefunden, welches dadurch gekennzeichnet ist, daß man Olefine der allgemeinen Formel II 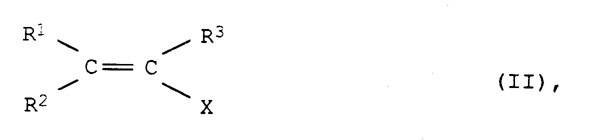 in der X, R¹, R², R³ und R⁴ die oben genannten Bedeutungen haben, mit H₂O₂ und/oder organischen Peroxiden an Titanzeolithen mit einer Kristallgröße von mehr als 1 µm und plättchenförmiger Kristallform bei Temperaturen von (-10) bis 180°C und Drücken von 1 bis 100 bar umsetzt.

Das erfindungsgemäße Verfahren läßt sich wie folgt durchführen:

Die erfindungsgemäße Reaktion der Olefine II mit H₂O₂ und/oder organischen Peroxiden zu den Oxiranen I kann durch Kontakt mit Titanzeolithen mit einer Kristallgröße von mehr als 1 µm und plättchenförmiger Kristallform bei Temperaturen von (-10) bis 180°C, bevorzugt 0 bis 100°C, besonders bevorzugt 20 bis 60°C und Drücken von 1 bis 100 bar, bevorzugt 1 bis 30 bar diskontinuierlich oder kontinuierlich in einem "Batch"-Reaktor, Suspensionsreaktor oder einem Festbettreaktor ausgeführt werden, wobei man einphasig aber auch zweiphasig arbeiten kann.

Als organische Peroxide eignen sich beispielsweise organische Hydroperoxide wie tert.-Butylhydroperoxid und Cyclohexylhydroperoxid.

Das Molverhältnis von H₂O₂ und/oder organischem Peroxid zum Olefin I liegt zwischen 0,2 : 1 bis 10 : 1 , bevorzugt zwischen 0,5 : 1 bis 3 : 1 , besonders bevorzugt zwischen 0,8 : 1 bis 2 : 1. Besonders vorteilhaft ist der Einsatz von wäßrigem H₂O₂.

Als Lösungsmittel kommen bei dem erfindungsgemäßen Verfahren bevorzugt polare Verbindungen wie Wasse, Alkohole, Ketone, Ether, Glykole oder Carbonsäuren in Frage, deren Zahl der Kohlenstoffatome kleiner gleich 6 ist. Auch Lösungsmittelgemische können vorteilhaft eingesetzt werden. Insbesondere finden Wasser, Methanol, Butanol, tert.-Butanol, Aceton, Methylethylketon, Methylisopropylketon, Essigsäure, Propionsäure und Glykol Verwendung. Aber auch unpolare Kohlenwasserstoffe lassen sich einsetzen als Lösungsmittel. Beispiele hierfür sind Cyclohexan, Xylol, Toluol und Benzol.

Wegen der gegebenenfalls bestehenden Temperaturempfindlichkeit sowohl der Olefine II als auch der erhaltenen Oxirane I, sollten die Reaktionsparameter nur in relativ engen Grenzen variiert werden.

Die Katalysatoren sind regenerierbar und lassen sich erneut einsetzen bzw. Ansätze können "batch"weis mit ein und demselben Katalysator ohne Zwischenregenerierung durchgeführt werden.

Zeolithe sind kristalline Aluminosilikate, die eine hochgeordnete Struktur mit einem starren dreidimensionalen Netzwerk von SiO₄- und AlO₄-Tetraedern besitzen, die durch gemeinsame Sauerstoffatome verbunden sind. Das Verhältnis der Si- und Al-Atome zu Sauerstoff beträgt 1 : 2 (siehe Ullmans Encyclopädie d. techn. Chemie, 4. Auflage, Band 24, Seite 575 bis 578 (1983)). Die Elektrovalenz der Aluminium enthaltenden Tetraeder ist durch Einschluß von Kationen in den Kristall, z. B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

Die Zeolithe werden zumeist in der aciden H-Form oder neutralen Alkali-Form angewendet. In den Zeolithen können anstelle von Aluminium auch andere Elemente, wie B, Ga, Fe, Cr, V, As, Sb, Bi oder Be oder deren Gemische in das Gitter eingebaut werden, oder das Silicium kann durch ein anderes vierwertiges Element, wie Ge, Ti, Zr, Hf ersetzt werden.

Entsprechend ihrer Struktur werden Zeolithe in verschiedene Gruppen unterteilt.

Für das erfindungsgemäße Verfahren verwendet man vorteilhaft Zeolithe vom Pentasiltyp. Diese haben als Grundbaustein einen aus SiO₄-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen.

Diese Zeolithe können verschiedene chemische Zusammensetzungen aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische. Der Aluminosilikatzeolith wird z.B. aus einer Aluminiumverbindung, vorzugsweise Al(OH)₃ oder Al₂(SO₄)₃ und einer Siliciumkomponente, vorzugsweise hochdispersem Siliciumdioxid in wäßriger Aminlösung, insbesondere in Polyaminen, wie 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Hierzu gehören auch die isotaktischen Zeolithe nach EP-A-34 727 und EP-A-46 504. Die erhaltenen Aluminosilikatzeolithe weisen je nach Wahl der Ausgangsstoffmengen ein SiO₂/Al₂O₃-Verhältnis von 10 bis 40 000 auf. Derartige Aluminosilikatzeolithe kann man auch in etherischem Medium, wie Diethylenglykoldimethylether, in alkoholischem Medium, wie Methan bzw. 1,4-Butandiol oder in Wasser synthetisieren. Barosilikatzeolithe lassen sich in analoger Weise herstellen.

Für das erfindungsgemäße Verfahren sind besonders vorteilhaft die Titanzeolithe mit einer Kristallgröße von mehr als 1 µm und plättchenförmiger Kristallform (MFI-Struktur).

Aus US-A-3 329 481 sind Materialien bekannt, bei denen im Silikatgitter anstelle des Si(IV) Titan als Ti(IV) sein soll. Diese Titanzeolithe, insbesondere solche mit einer Kristallstruktur vom MFI-Typ (vgl. Meier, Olson, a.a.O), sowie Möglichkeiten zu ihrer Herstellung sind beschrieben beispielsweise in US-A-4 410 501, EP-A-311 983, US-A-4 666 692, DE-A-30 47 798 oder in BE-A-10 01 038. Titanhaltige Zeolithe mit anderen Strukturen kennt man aus EP-A-405 978. Außer Silizium und Titan können solche Materialien auch zusätzliche Elemente wie Gallium (EP-A-266 825), Bor (US-A-4 666 692) oder geringe Mengen an Fluor enthalten (EP-A-292 363).

Titanzeolithe mit MFI-Struktur sind dafür bekannt, daß sie über ein bestimmtes Muster bei der Bestimmung ihrer Röntgenbeugungsaufnahmen, sowie zusätzlich über eine Gerüstschwingungsbande im IR-Bereich bei etwa 950 cm⁻¹ identifiziert werden können (DE-A-30 47 798) und sich damit von Alkalititanaten oder kristallinen und amorphen TiO₂-Phasen unterscheiden.

Typischerweise stellt man die vorgenannten Titanzeolithe dadurch her, daß man eine wässrige Mischung aus einer SiO₂-Quelle, einem Titandioxid und einer stickstoffhaltigen organischen Base, wie etwa Tetrapropylammoniumhydroxid, gegebenenfalls noch unter Hinzufügen von Alkali in einem Druckbehälter unter erhöhter Temperatur im Zeitraum mehrerer Stunden oder weniger Tage umsetzt, wobei das kristalline Produkt entsteht. Dieses wird abfiltriert, gewaschen, getrocknet und zur Entfernung der organischen Stickstoffbase bei erhöhter Temperatur gebrannt. In dem so erhaltenen Pulver liegt das Titan zumindest teilweise innerhalb des Zeolithgerüsts in wechselnden Anteilen mit vier-, fünf- oder sechsfacher Koordination vor (Behrens, et al.; J. Chem. Soc., Chem. Commun. 1991, S. 678 bis 680). Zur Verbesserung des katalytischen Verhaltens kann sich noch eine mehrmalige Waschbehandlung mit schwefelsaurer Wasserstoffperoxidlösung anschließen, worauf das Titanzeolith-Pulver erneut getrocknet und gebrannt werden muß, wie es etwa in EP-A-267 362 beschrieben wird. Der pulverförmige Titanzeolith muß nun abschließend unter Zusatz eines geeigneten inerten Binders in einem Formgebungsschritt verarbeitet werden, um ihn als Katalysator in einer handhabbaren Form verfügbar zu machen. Eine Methode hierzu wird in EP-A-200 260 aufgezeigt. DE-A-41 38 155 beschreibt die Kristallisation von Titansilikaten mit Zeolithstruktur durch eine hydrothermale Umsetzung einer SiO₂-Quelle mit einer Titankomponente in Gegenwart wässriger Lösungen von schwach konzentriertem Tetraalkylammoniumhalogeniden und zusätzlich Ammoniak. Dabei ermöglicht es das erfindungsgemäße Verfahren, daß man die entstehenden Titanzeolith-Kristalle in hoher Ausbeute und mit einer Kristallgröße von mehr als 1 µm direkt in Form plättchenförmiger, weitestgehend alkalifreier, großer Primärkristallite erhält, die aufgrund ihrer Teilchengröße ohne weitere Verformungsschritte als Katalysatoren zur Umsetzung organischer Moleküle und zwar insbesondere in Wirbelschicht-, Rieselbett- oder Suspensionsfahrweisen verwendet werden können.

Der Vorteil dieser letzteren Methode ist, daß man auf den Einsatz von teurem Tetrapropylammoniumhydroxid verzichten kann, wenn man stattdessen geringe Mengen billiger Tetrapropylammoniumhalogenide, beispielsweise Tetrapropylammoniumbromid verwendet, im folgenden als TPABr bezeichnet, und dabei ein molares Verhältnis von TPABr/SiO₂ von 0,042 : 1 bis 0,2 : 1, vorzugsweise aber von 0,042 : 1 bis 0,15 : 1 in der Reaktionsmischung einhält. Als vorteilhaft hat es sich gezeigt, daß man die Kristallisation der Titansilikate mit Zeolithstruktur hydrothermal bei Temperaturen von 100°C bis 250°C, besonders bei 120 bis 200°C und insbesondere bei 150°C bis 190°C durchführt. Dabei ist es angebracht, daß man dabei ein molares Verhältnis von, als wäßrige Lösung eingesetztem Ammoniak, zu SiO₂ von 10 : 1 bis 1 : 1, besser von 6 : 1 bis 1,5 : 1 und besonders bevorzugt von 5 : 1 bis 3 : 1 einhält.

Katalytisch verwendbare Titansilikat-Zeolithe erhält man dadurch, daß man die Titankomponente in Form einer löslichen, wässrigen oder wässrig-alkoholischen Peroxotitanatverbindung in der vorbeschriebenen Weise bei der hydrothermalen Umsetzung der Reaktionsmischung zusetzt. Dies gelingt dadurch, daß man als molare Verhältnisse der Reaktionsmischung im Bereich von SiO₂/TiO₂ mit 10 bis 1500, und insbesondere von 10 bis 250, bevorzugt aber von 10 bis 100 arbeitet und/oder dabei eine Verdünnung von SiO₂/H₂O mit 0,07 bis 0,025, bevorzugt von 0,05 bis 0,04 beachtet.

Dieses alkalifreie Verfahren macht es möglich, daß darüber hinaus das Material nach einer Temperaturbehandlung bei 350 bis 600°C, bevorzugt bei 400 bis 550°C und insbesondere vorteilhafterweise bei 450 bis 500°C direkt und ohne zusätzlichen Ionenaustausch und insbesondere aufgrund der Kristallgröße von mehr als 1 µm und der plättchenförmigen Kristallform ohne weitere Formgebung in einer katalytisch wirksamen Form vorliegt und als Katalysator verwendet werden kann.

Die so hergestellten, aminfreien Titansilikate mit MFI-Struktur verfügen in ihrem IR-Spektrum über eine charakteristische Bande bei 950 bis 960 cm⁻¹, wohingegen die noch das Tetrapropylammonium enthaltenden, aber ansonsten erfindungsgemäß hergestellten Titansilikate diese Bande erst nach einer Temperaturbehandlung bei 350 bis 600°C, insbesondere bei Temperaturen zwischen 450 und 550°C, aufweisen. Eine Charakterisierung der Titansilikate ist auch über ihr spezifisches Röntgendiffraktogramm möglich, wobei die aminfreien Titansilikate ein der orthohombischen MFI-Struktur entsprechendes Diffraktogramm haben.

Zeolithpulver kann man nach ihrer Herstellung einem Verformungsschritt unterwerfen. Hierdurch erhält man Formkörper unterschiedlicher Gestalt, Tabletten, Ringe, Stränge, Sterne, Kleeblattformen, etc. Zeolithe können nach ihrer Isolierung, Trocknung bei 100 bis 160°C, vorzugsweise 110°C und Calcinierung bei 450 bis 550°C, vorzugsweise 500°C, mit einem Bindemittel im Verhältnis 90 : 10 bis 40 : 60 Gew.-% zu Strängen oder Tabletten verformt werden. Als Bindemittel eignen sich verschiedene Aluminiumoxide, insbesondere Boehmit, amorphe Aluminosilikate mit einem SiO₂/Al₂O₃-Verhältnis von 25 : 75 bis 90 : 5, insbesondere 75 : 25, Siliciumdioxid, insbesondere hochdisperses SiO₂, Gemische aus hochdispersem SiO₂ und hochdispersem Al₂O₃, TiO₂, ZrO₂ sowie Ton. Nach der Verformung werden die Extrudate der Preßlinge bei 110°C/16 h getrocknet und bei 500°C/16 h calciniert.

Die Substituenten X, R¹, R², R³ und R⁴ in den Verbindungen I und II haben folgende Bedeutungen:
- X: -COOH,
-COOR⁴,
-CN,
- R¹,R²,R³,R⁴: - unabhängig voneinander
- C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
- Aryl wie Phenyl, 1-Naphthyl, 2-Naphthyl, 9-Naphthyl und Biphenyl, bevorzugt Phenyl,
- C₇- bis C₁₂-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl,
- R¹,R²,R³: - unabhängig voneinander
- Wasserstoff.

Als Edukte werden z.B. die nachfolgend genannten Carbonsäuren und Carbonsäurederivate eingesetzt: Acrylsäure, Methacrylsäure, Crotonsäure, Methylacrylat, Ethylacrylat, Methylmethacrylat, Ethylmethacrylat, Methylcrotonat, Acrylnitril, Zimtsäuremethylester, etc. Oxirane sind aufgrund ihrer hohen Reaktivität wertvolle Zwischenprodukte bei der Synthese zahlreicher Verbindungsklassen. Substitu- ierte Oxirane wie z.B. Derivate der Glycidsäure können u.a. zu 2-Hydroxypropionsäurederivaten umgesetzt werden. Die Darstellung solcher Verbindungen sowie ihre Verwendung als Komplexbildner in Wasch- und Reinigungsmitteln ist z.B. in EP-A-287 846 beschrieben.

### Beispiele

### Herstellungsbeispiel 1

Dieses Beispiel beschreibt die Herstellung eines nicht erfindungsgemäßen Titansilikats mit MFI-Struktur, wie es nach dem Stand der Technik in EP-A-376 453 beschrieben ist, genannt Katalysator A.

30 ml Tetraethylorthotitanat wurden unter Rühren (300 U/min) innerhalb von 15 Minuten in 375 ml deionisiertes Wasser getropft, welches zuvor auf 2°C abgekühlt wurde. Danach wurde mit 360 ml kalter Wasserstoffperoxidlösung (30 Gew.-%) versetzt, worauf sich eine orangerote Lösung bildete, die für die Dauer von 2 Stunden gerührt wurde. Danach wurden 625 ml wäßrige Tetrapropylammoniumhydroxidlösung (20-Gew.-%) und nach einer weiteren Stunde 100 g kolloidale Silicasol®-Lösung (40 Gew.-% SiO₂, Ludox AS®-40) zugegeben. Diese Mischung wurde über Nacht bei Raumtemperatur aufbewahrt, am nächsten Tag bei 80°C unter Rühren (300 U/min) für die Dauer von 7 Stunden gekocht, in einen 2 l fassenden Druckrührbehälter eingefüllt und für die Dauer von 240 Stunden bei 175°C zur Reaktion gebracht.

Das erkaltete Reaktionsgemisch wurde abfiltriert, der Filterkuchen mehrmals mit deionisiertem Wasser neutralgewaschen, bei 120°C über Nacht getrocknet und abschließend bei 550°C in Luftatmosphäre kalziniert.

Bezogen auf eingesetztes SiO₂ betrug die Ausbeute an Titanzeolith 93 %. Nach dem Röntgenbeugungsmuster handelt es sich um reinen Titanzeolith vom Silikalittyp.

### Herstellungsbeispiele 2 und 3

Dieses Synthesebeispiel beschreibt die Herstellung der Katalysatoren B und C durch Wechsel von Tetrapropylammoniumhydroxid zu Tetrapropylammoniumbromid unter gleichzeitiger Verwendung von Ammoniaklösung.

In einem mit Rührer und Rückflußkühler versehenen Glaskolben werden 45,1 g deionisiertes Wasser auf 5°C abgekühlt. Dazu tropft man innerhalb von 15 Minuten 6,9 g Tetraisopropylorthotitanat und 81,4 g Wasserstoffperoxidlösung (30 Gew.-%). Zu der entstandenen orangeroten Lösung fügt man 211 g einer Ammoniaklösung (25 Gew.-%) und läßt den entstandenen Ansatz sich über Nacht auf Raumtemperatur erwärmen. Abschließend wird für die Dauer von 3 Stunden auf 80°C unter Rühren erwärmt. Eventuell eintretender Gewichtsverlust wird durch Zugabe einer entsprechenden Menge an Ammoniaklösung ausgeglichen.

Von dieser so vorbereiteten Lösung werden 156,8 g mit 56 g Tetrapropylammoniumhydroxidlösung (20 % in Wasser) und 52,8 g Ludox AS®-40 Silicasol innerhalb von 3 Minuten gemischt, in einen teflonausgekleideten Autoklavenbehälter eingefüllt und druckdicht verschlossen. Dies wird im folgenden als Katalysator B bezeichnet.

Weitere 156,5 g der eingangs vorbereiteten Lösung werden mit 14,7 g Tetrapropylammoniumbromid in 44,8 g Wasser und 53,1 g Ludox AS-40 Silicasol® innerhalb von 3 Minuten gemischt, in einen teflonausgekleideten Autoklavenbehälter eingefüllt, und druckdicht verschlossen. Dies wird im folgenden als Katalysator C bezeichnet.

Die Katalysatoren B und C werden getrennt voneinander bei einer Temperatur von 183 bis 185°C innerhalb von 192 Stunden zur Reaktion gebracht. Die kristallinen Reaktionsprodukte werden abfiltriert, neutralgewaschen, getrocknet und an Luft bei 500°C für die Dauer von 5 Stunden kalziniert.

Die Eigenschaften beider Ansätze sind nachfolgend in Tabelle 1 gegenübergestellt.

**Tabelle 1**

| Katalysator | Ausbeute | Si/Ti molar | Kalium Gew.-% | Größe µm | Form |
|---|---|---|---|---|---|
| B* | 96 % | 36 | 0,89 | 5 | globulär |
| C | 95 % | 36 | 0,0012 | 26 | plättchenförmig |

| | | | | | |
|---|---|---|---|---|---|
| * nicht erfindungsgemäß | | | | | |

### Herstellungsbeispiel 4

Beschreibt die Herstellung von Katalysator D unter Verwendung einer pyrogenen Kieselsäure als Silicaquelle. In einem mit Rührer und Rückflußkühler versehenen Glaskolben werden 45,2 g deionisiertes Wasser auf 5°C abgekühlt. Dazu tropft man innerhalb von 15 Minuten 6,9 g Tetraisopropylorthotitanat und 81,5 g Wasserstoffperoxidlösung (30 Gew.-%). Zu der entstandenen orangeroten Lösung fügt man 211,0 g einer Ammoniaklösung (25 Gew.-%) und läßt den entstandenen Ansatz sich über Nacht auf Raumtemperatur erwärmen. Abschließend wird für die Dauer von 3 Stunden auf 80°C unter Rühren erwärmt. Eventuell eintretender Gewichtsverlust wird durch Zugabe einer entsprechenden Menge an Ammoniaklösung ausgeglichen. Die so hergestellte Lösung wird mit 14,7 g Tetrapropylammoniumbromid, 75,5 g Wasser und 22,1 g Aerosil®-200 (pyrogene Kieselsäure) in einen Stahlautoklaven eingefüllt.

Im Laufe von 168 Stunden bei einer Temperatur von 185°C wird die Reaktionsmischung umgesetzt, nach dem Abkühlen das kristalline Produkt abfiltriert, neutralgewaschen, getrocknet und bei 500°C innerhalb 5 Stunden in Luftatmosphäre kalziniert.

Das Produkt zeigt das typische Röntgendiffraktogramm des TS-1 Titansilikalits. Die Kristalle haben eine gleichförmige Größe von ca. 8 µm mit einem plättchenförmigem Habitus. Die IR-Aufnahme der Probe zeigt deutlich eine scharfe Bande bei 960 cm⁻¹ (vgl. Abb. 5). Die chemische Analyse ergibt ein molares Verhältnis im Produkt von Si/Ti = 37,7. Die Verunreinigungen durch Alkali betrugen nur 0,0015 Gew.-% Natrium und 0,0045 Gew.-% an Kalium. Bezogen auf eingesetztes SiO₂ ergab sich eine Ausbeute an kristallinem, kalzinierten Produkt von 90,3 %.

Die mit diesen Katalysatoren durchgeführten Umsetzungen sind in den nachfolgenden Beispielen aufgelistet.

### Beispiel 1 bis 20

Die Reaktionen werden "batch"weise in einem gläsernen Rührautoklaven mit getöntem Glas (Volumen 250 ml) durchgeführt. Das Edukt wird im Lösungsmittel gelöst und zusammen mit dem Katalysator unter Rühren auf Reaktionstemperatur gebracht (vgl. Tabelle 2). Dann wird mit einer Pumpe die Peroxidlösung in 15 min zudosiert und anschließend die in Tabelle 2 genannte Zeit weitergerührt. Nach dem Abkühlen der Reaktionsmischung wird der Katalysator abfiltriert und die Lösung durch GC analysiert. In den Lösungen konnte kein Peroxid mehr nachgewiesen werden. Als Nebenprodukt trat manchmal wenig der entsprechenden α-Hydroxyverbindung auf (also z.B. beim Einsatz von Acrylsäuremethylester 2-Hydroxypropionsäuremethylester).

Die Versuchsergebnisse sind in Tabelle 2 zusammengestellt.

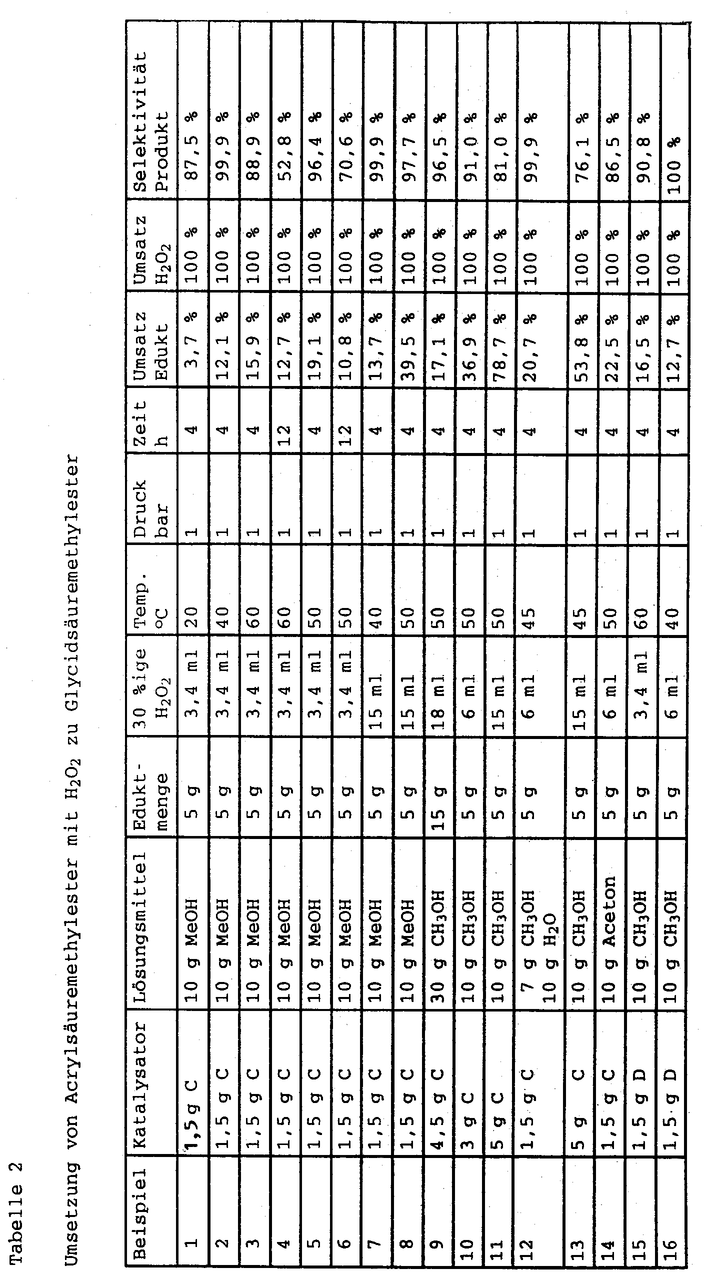

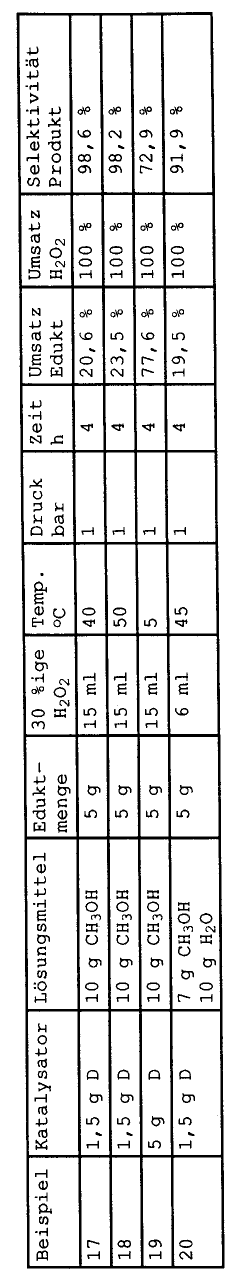

### Beispiel 21 und 22

Die Epoxidierung von Crotonsäuremethylester bei 50°C führt zum gewünschten Produkt mit 97 % Selektivität bei 4,5 % Umsatz Edukt und 100 % Umsatz H₂O₂ am Katalysator C (Beispiel 21). Am Katalysator D (Beispiel 22) sind bei 40°C die Werte 62,8 % Selektivität bei 3,1 % Umsatz Edukt und vollständigem H₂O₂-Umsatz. Bei diesen Versuchen beträgt die Katalysatormenge 1,5 g, die Eduktmenge 5,0 g, die Methanolmenge 10 g, die H₂O₂(30 %ig)-Menge 3,4 ml und die Reaktionszeit 4 h.

### Beispiel 23 bis 25

Die Epoxidierung von Methacrylsäuremethylester bei 50°C an Katalysator C (Beispiel 23) führt zum gewünschten Produkt mit 90,8 % Selektivität bei 6,5 % Umsatz Edukt und vollständigem H₂O₂-Umsatz. Bei 40°C werden 62,4 % Selektivität bei 3,9 % Eduktumsatz gefunden (Beispiel 24). Wird die Reaktion bei 40°C an Katalysator D (Beispiel 25) ausgeführt, so wird das Oxiran mit 71,8 % Selektivität bei 4,6 % Edukt- und 100 % H₂O₂-Umsatz gebildet.

Bei diesen Versuchen beträgt die Katalysatormenge 1,5 g, die Eduktmenge 5,0 g, die Methanolmenge 10 g, die H₂O₂(30 %ig)-Menge 3,4 ml und die Reaktionszeit 4 h.

### Beispiel 26

Der Katalysator C wird mehrmals hintereinander für die Umsetzung von Acrylsäuremethylester im batch-Verfahren eingesetzt. Die Bedingungen sind 4,5 g Katalysator, 30 g CH₃OH, 15 g Edukt, 18 ml 30%ige H₂O₂, 50°C, 1 bar und 4 h Laufzeit. Die Ergebnisse sind in Tabelle 3 zusammengestellt. Der H₂O₂-Umsatz ist immer vollständig.

**Tabelle 3**

| Ansatz | 1 | 2 | 3 | 4 | 5 | 6 |
|---|---|---|---|---|---|---|
| Umsatz Edukt | 18,5 | 18,5 | 15,8 | 18,6 | 18,6 | 18,6 |
| Selektivität | 99,9 | 99,9 | 99,9 | 99,9 | 99,9 | 99,9 |

## Patentansprüche

1. Verfahren zur Herstellung von Oxiranen der allgemeinen Formel I 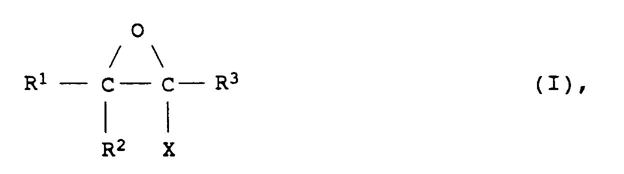 in der
X -COOH, -COOR⁴ oder -CN,
R¹,R²,R³,R⁴ C₁- bis C₈-Alkyl, C₃- bis C₈-Cycloalkyl, Aryl und C₇- bis C₁₂-Aralkyl oder
R¹,R²,R³ Wasserstoff bedeuten,
dadurch gekennzeichnet, daß man Olefine der allgemeinen Formel II 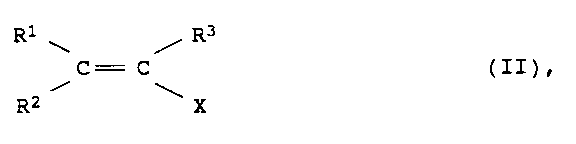 in der X, R¹, R², R³ und R⁴ die oben genannten Bedeutungen haben, mit H₂O₂ und/oder organischen Peroxiden an Titanzeolithen mit einer Kristallgröße von mehr als 1 µm und plättchenförmiger Kristallform bei Temperaturen von (-10) bis 180°C und Drücken von 1 bis 100 bar umsetzt.

2. Verfahren zur Herstellung von Oxiranen I nach Anspruch 1, dadurch gekennzeichnet, daß man Titanzeolithe mit Pentasilstruktur verwendet.

3. Verfahren zur Herstellung von Oxiranen I nach AnSpruch 1, dadurch gekennzeichnet, daß man die Umsetzung bei Temperaturen von 0 bis 100°C und Drücken von 1 bis 30 bar durchführt.

4. Verfahren zur Herstellung von Oxiranen I nach Anspruch 1, dadurch gekennzeichnet, daß man die Umsetzung bei Temperaturen von 20 bis 60°C und Drücken von 1 bis 30 bar durchführt.

## Claims

1. A process for the preparation of oxiranes of the formula I 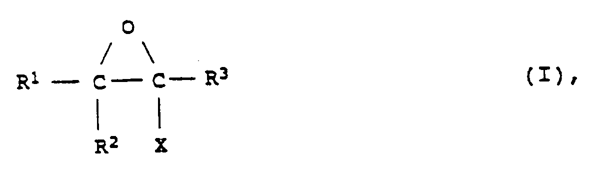 where
X is -COOH, -COOR⁴ or -CN,
R¹,R²,R³ and R⁴ are each C₁-C₈-alkyl, C₃-C₈-cycloalkyl, aryl or C₇-C₁₂-aralkyl or
R¹,R² and R³ are each hydrogen,
which comprises reacting olefins of the formula II 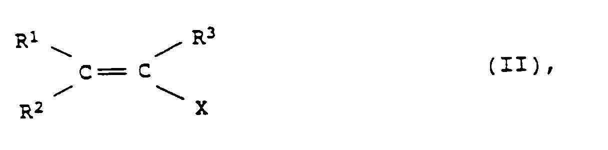 where X, R¹, R², R³ and R⁴ have the abovementioned meanings, with H₂O₂ or organic peroxides over titanium zeolites having a crystal size of more than 1 µm and a tabular crystal habit at from -10 to 180°C and from 1 to 100 bar.

2. A process for the preparation of oxiranes I as claimed in claim 1, wherein titanium zeolites having a pentasil structure are used.

3. A process for the preparation of oxiranes I as claimed in claim 1, wherein the reaction is carried out at from 0 to 100°C and from 1 to 30 bar.

4. A process for the preparation of oxiranes I as claimed in claim 1, wherein the reaction is carried out at from 20 to 60°C and from 1 to 30 bar.

## Revendications

1. Procédé de préparation d'oxirannes de la formule générale I 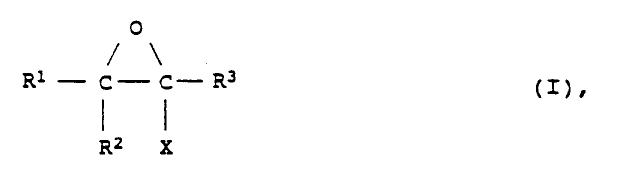 dans laquelle
X représente -COOH, -COOR⁴ ou -CN,
R¹, R², R³, R⁴ représentent chacun un radical alkyle en C₁ à C₈, cycloalkyle en C₃ à C₈, aryle et aralkyle en C₇ à C₁₂, ou bien
R¹, R², R³ représentent chacun un atome d'hydrogène,
caractérisé en ce que l'on fait réagir des oléfines de la formule générale II 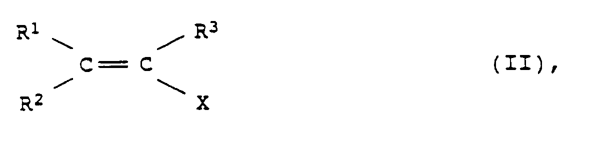 dans laquelle X, R¹, R², R³ et R⁴ possèdent les significations qui leur ont été attribuées ci-dessus, avec H₂O₂ et/ou des peroxydes organiques sur des zéolites au titane, d'un calibre des cristaux supérieur à 1 µm et de forme cristalline en plaquettes, à des températures de -10 à 180°C et sous des pressions de 1 à 100 bars.

2. Procédé de préparation d'oxirannes I suivant la revendication 1, caractérisé en ce que l'on utilise des zéolites au titane à structure de pentasile.

3. Procédé de préparation d'oxirannes I suivant la revendication 1, caractérisé en ce que l'on entreprend la réaction à des températures de 0 à 100°C et sous des pressions de 1 à 30 bars.

4. Procédé de préparation d'oxirannes I suivant la revendication 1, caractérisé en ce que l'on entreprend la réaction à des températures de 20 à 60°C et sous des pressions de 1 à 30 bars.
